# EUROPEAN PATENT SPECIFICATION

(11) **EP 2 692 391 B1**
(45) Date of publication and mention of the grant of the patent: **27.05.2020**
(21) Application number: 13178419.1
(22) Date of filing: 29.07.2013
(51) Int. Cl.: A61M 25/10

(54) **Catheter for the treatment of bodily passages**
Katheter zur Behandlung von Körperdurchgängen
Cathéters pour le traitement de passages corporels

(30) Priority: 30.07.2012 US 201261677156 P
(43) Date of publication of application: 05.02.2014
(73) Proprietor: Cook Medical Technologies LLC, Bloomington, IN 47404 (US)
(72) Inventor: Schaeffer, Darin, Indiana 47401 (US); Kost, Karen, Quebec H3Y 2S2 (CA)
(74) Representative: Mathys & Squire

(56) References cited:
- EP-A2- 0 835 673
- WO-A1-2009/036135
- WO-A1-2010/024871
- WO-A1-2010/065030
- CN-A- 102 600 546
- US-A- 5 873 852
- US-A1- 2001 041 859
- US-A1- 2004 054 351
- US-A1- 2004 064 093
- US-A1- 2010 185 146

## Description

### Field

The disclosure relates generally to medical devices. More particularly, the disclosure relates to catheters useful in the treatment of bodily passages, such as an airway, sinus cavity, or sinus passage. The disclosure also relates to methods of treating bodily passages.

### Background

To treat stenosis - the narrowing of a bodily passage - physicians frequently dilate the bodily passage using a balloon catheter. In these procedures, the balloon catheter is navigated to a point of treatment and inflated to dilate the narrowed bodily passage. However, in some cases the presence of scar tissue or lesions - build up - in the bodily passage introduces complexities to the procedure and prevents efficient dilation of the bodily passage. Therefore, to facilitate efficient dilation of a bodily passage that contains build up, it is necessary to first cut or break the build up. This can be accomplished using conventional procedures, such as by breaking up the build up using a scalpel, or laser, and then introducing a smooth surfaced balloon catheter into the bodily passage to effectuate dilation. Alternatively, a cutting balloon catheter can be used to both break up the build up and dilate the bodily passage.

Both of these approaches, however, fail to provide a mechanism for introducing a medication at the point of treatment during the dilation process. For example, once one of these approaches has been completed, it is often necessary, or otherwise desirable, to apply a medication or other therapeutic agent at the point of treatment to prevent further scar tissue build up or to promote healing. This requires the introduction of an applicator, or other device, into the bodily passage to apply the medication at the point of treatment. Thus, multiple devices must be advanced separately into the bodily passage to accomplish dilation and delivery of the medication, which increases the complexity and time required to complete the procedure.

Reference is directed to WO2010065030 which discloses a balloon catheter is provided that may be used to dilate hardened regions of a stenosis on a vessel wall. The balloon catheter is provided with cutting elements that extend along a surface of a balloon. A bioactive is delivered to the vessel wall upon dilation of the balloon and may pass through holes in the cutting elements. Reference is also directed to CN102600546 which discloses a balloon catheter for delivering a therapeutic and/or diagnostic agent to tissue is described including an outer balloon having a wall with an opening there through and an inner surface, an inner balloon disposed in the outer balloon, enclosing an inflation chamber and having an outer surface defining a space between the outer surface of the inner balloon and the inner surface of the outer balloon, a catheter having a first lumen in fluid communication with the space between the inner balloon and the outer balloon for supplying the agent thereto, and a second lumen through which fluid is supplied to the inflation chamber for inflating the inner balloon to urge the agent out of the opening in the outer balloon. The outer balloon and/or the inner balloon comprises at least one protrusion for directing the agent. Reference is further directed to WO2010065030 which discloses apparatus for treating tissue by delivering at least one therapeutic agent into the tissue. The apparatus comprises a catheter and a balloon member disposed on a distal region of the catheter. A plurality of pockets are disposed on the balloon member and a plurality of needles are associated with each of the plurality of pockets. The plurality of needles are configured to engage tissue when the balloon is in the inflated state, and further are configured to disperse a therapeutic agent from an associated pocket into the tissue when the balloon is in the inflated state. US 5,873,852A discloses a device for injecting fluid into a treatment area of a vessel wall.

Therefore, a need exists for improved medical devices and methods for dilating a bodily passage and delivering a medication.

### Summary

References to "embodiments" throughout the description which are not under the scope of the appended claims merely represent possible exemplary executions and are therefore not part of the present invention.

Various exemplary medical devices are described.

A first exemplary medical device comprises an elongate member, a first balloon, and a second balloon. The elongate member has an elongate member proximal end, an elongate member distal end, and defines an infusion lumen and an inflation lumen. The first balloon is attached to the elongate member and is adapted to move between a deflated configuration and an inflated configuration. The first balloon comprises a first balloon wall that defines an infusion chamber and at least one protuberance that extends radially outward from the first balloon wall. The infusion chamber is in communication with the infusion lumen. The at least one protuberance defines at least one protuberance pore in communication with the infusion chamber such that fluid introduced into the infusion chamber can pass through the at least one protuberance pore. The second balloon is attached to the elongate member and is disposed radially inward of the first balloon. The second balloon is adapted to move between a deflated configuration and an inflated configuration and comprises a second balloon wall that defines an inflation chamber in communication with the inflation lumen.

A second exemplary medical device comprises an elongate member, a first balloon, and a second balloon. The elongate member has an elongate member proximal end, an elongate member distal end, and defines an infusion lumen and an inflation lumen. The first balloon is attached to the elongate member and is adapted to move between a deflated configuration and an inflated configuration. The first balloon comprises a first balloon wall that defines an infusion chamber and a plurality of protuberances that extends radially outward from the first balloon wall. The infusion chamber is in communication with the infusion lumen. Each protuberance of the plurality of protuberances defines a plurality of protuberance pores in communication with the infusion chamber such that fluid introduced into the infusion chamber can pass through each protuberance pore of the plurality of protuberance pores. The second balloon is attached to the elongate member and is disposed radially inward of the first balloon. The second balloon is adapted to move between a deflated configuration and an inflated configuration and comprises a second balloon wall that defines an inflation chamber in communication with the inflation lumen.

Methods of treatment are also described.

An exemplary method of treatment comprises a step of inserting a medical device having a medical device proximal end and a medical device distal end into a bodily passage such that the medical device distal end is disposed in the bodily passage. The medical device comprises an elongate member, a first balloon, and a second balloon. The elongate member has an elongate member proximal end, an elongate member distal end, and defines an infusion lumen and an inflation lumen. The first balloon is attached to the elongate member and is adapted to move between a deflated configuration and an inflated configuration. The first balloon comprises a first balloon wall defining an infusion chamber and at least one protuberance that extends radially outward from the first balloon wall. The infusion chamber is in communication with the infusion lumen. The at least one protuberance defines at least one protuberance pore in communication with the infusion chamber such that fluid introduced into the infusion chamber can pass through the at least one protuberance pore. The second balloon is attached to the elongate member and disposed radially inward of the first balloon. The second balloon is adapted to move between a deflated configuration and an inflated configuration and comprises a second balloon wall defining an inflation chamber in communication with the inflation lumen. Another step comprises navigating the medical device distal end to a point of treatment within the bodily passage. Another step comprises passing a fluid into the inflation chamber to move the second balloon to the inflated configuration such that a portion of the first balloon contacts the wall of the bodily passage. Another step comprises continuing to pass fluid into the inflation chamber such that the at least one protuberance damages the wall of the bodily passage. Another step comprises passing a medication through the infusion lumen and into the infusion chamber such that the medication passes through the at least one protuberance pore. Another step comprises stopping the passing of a medication through the infusion lumen and into the infusion chamber. Another step comprises removing a portion of the fluid from the inflation chamber. Another step comprises withdrawing the medical device distal end from said bodily passage.

Additional understanding of the exemplary medical devices and methods can be obtained by review of the detailed description, below, and the appended drawings.

### Brief Description of the Figures

Figure 1 is a side view of an exemplary catheter.
Figure 2 is a distal end view of the catheter illustrated in Figure 1.
Figure 3 is a sectional view of the catheter illustrated in Figures 1 and 2, taken along line 3-3 in Figure 1.
Figure 4 is a sectional view of the catheter illustrated in Figures 1, 2, and 3, taken along line 4-4 in Figure 2.
Figure 5 is a side view of the distal end of an alternative catheter.
Figure 6 is a side view of the distal end of a second alternative catheter.
Figure 7 is a side view of the distal end of a third alternative catheter.
Figure 8 is a side view of the distal end of a fourth alternative catheter.
Figure 9 is a side view of the distal end of a fifth alternative catheter.
Figure 10 is a side view of the distal end of a sixth alternative catheter.
Figure 11 is a side view of the distal end of a seventh alternative catheter.
Figure 12 is a flowchart representation of an exemplary method of treatment.

### Detailed Description

The following detailed description and the appended drawings describe and illustrate various exemplary medical devices and methods. The description and drawings are exemplary in nature and are provided to enable one skilled in the art to make and use one or more exemplary medical devices, and/or practice one or more exemplary methods. They are not intended to limit the scope of the claims in any manner.

The use of "e.g.," "etc.," "for instance," "in example," and "or" and grammatically related terms indicates non-exclusive alternatives without limitation, unless otherwise noted. The use of "optionally" and grammatically related terms means that the subsequently described element, event, feature, or circumstance may or may not be present/occur, and that the description includes instances where said element, event, feature, or circumstance occurs and instances where it does not. The use of "exemplary" refers to "an example of' and is not intended to convey a meaning of an ideal or preferred embodiment. The use of "attached" refers to the fixed, releasable, or integrated association of two or more elements and/or devices. Thus, the term "attached" includes releasably attaching or fixedly attaching two or more elements and/or devices. As used herein, the terms "proximal" and "distal" are used to describe opposing axial ends of the particular elements or features being described. The use of "bodily passage" or "body passage" refers to any passage within the body of an animal, including, but not limited to, humans, and includes elongate passages. The term "sinus passage" refers to the nasal passages, and includes, but is not limited to, eustachian tube(s), primary ostium, accessory ostium, and/or an opening defined by a ventilation tube. The term "airway" refers to any airway including, but not limited to, the nasopharynx, oropharynx, pharynx, trachea, bronchial tubes, esophagus, and/or lungs. The term "sinus cavity" refers to the frontal, ethmoid, sphenoid, and/or maxillary sinus. The term "medication" refers to any fluid, drug, agent, therapeutic agent (e.g., stem cells), and/or any other material used to treat a patient. The term "damage," "damaging," and grammatically related terms refers to shattering, cracking, fracturing, fragmenting, scoring, cutting, breaking, ripping, tearing, perforating, interacting, disrupting, expanding, penetrating, damaging, and/or puncturing.

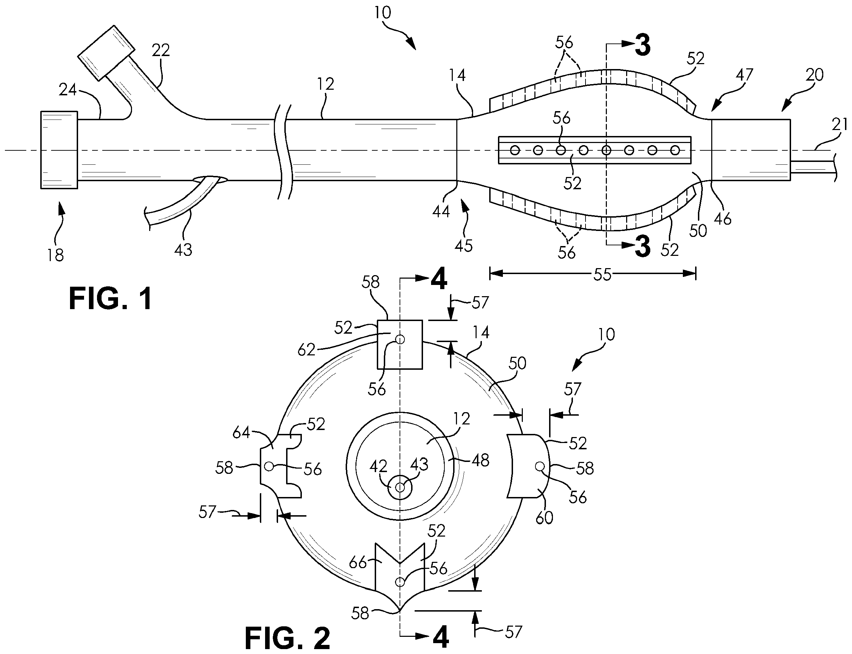

Figures 1, 2, 3, and 4 illustrate an exemplary catheter 10 comprising an elongate member 12, a first balloon 14, and a second balloon 16.

Elongate member 12 can have any suitable outside diameter and length, and skilled artisans will be able to select a suitable outside diameter and length for an elongate member according to a particular embodiment based on various considerations, including the desired bodily passage within which the catheter is intended to be used.

Elongate member 12 can be formed of any suitable material, and skilled artisans will be able to select a suitable material for an elongate member according to a particular embodiment based on various considerations, including the desired flexibility of the elongate member. Example materials considered suitable for an elongate member include, but are not limited to, biocompatible materials, materials that can be made biocompatible, metals such as stainless steel, titanium, nickel-titanium alloy (e.g., Nitinol), polymers, Pebax (Pebax is a registered trademark of Ato Chimie Corporation of Allee des Vosges, Courbevoie, France), nylon, polyethylene, polyurethane, silicone, coiled materials, and braided materials.

In the illustrated embodiment, elongate member 12 comprises an elongate member proximal end 18, an elongate member distal end 20, has an elongate member lengthwise axis 21, and defines an infusion port 22, an inflation port 24, an infusion lumen 26, an inflation lumen 28, and a guide wire lumen 30.

The infusion port 22 and inflation port 24 are disposed on a proximal portion of elongate member 12 and can include any suitable connector and/or adapter capable of attaching one or more devices to elongate member 12. Skilled artisans will be able to select a suitable connector and/or adapter to include on an infusion port and/or inflation port of an elongate member according to a particular embodiment based on various considerations, including the materials that form the elongate member. Example connectors and/or adapters considered suitable to include on an infusion port and/or inflation port of an elongate member include, but are not limited to, threaded connectors, Tuohy Borst adapters, luer lock connectors, and any other connector and/or adapter considered suitable for a particular application.

Infusion lumen 26 extends from a first infusion lumen opening 32 defined on infusion port 22 to a second infusion lumen opening 34 defined between elongate member proximal end 18 and elongate member distal end 20. Inflation lumen 28 extends from a first inflation lumen opening 36 defined on inflation port 24 to a second inflation lumen opening 38 defined between elongate member proximal end 18 and elongate member distal end 20. Guide wire lumen 30 extends between a first guide wire lumen opening 40 defined between elongate member proximal end 18 and elongate member distal end 20 to a second guide wire lumen opening 42 defined on elongate member distal end 20. Guide wire lumen 30 is adapted to receive a guide wire 43 and allow guide wire 43 such that guide wire 43 can pass through first guide wire lumen opening 40, guide wire lumen 30, and second guide wire lumen opening 42, and towards a point of treatment.

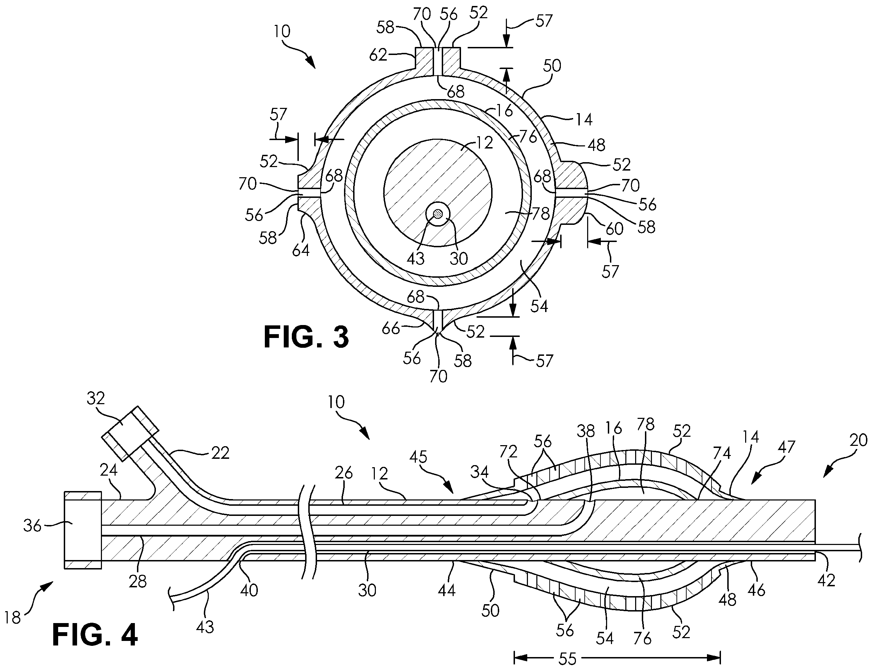

While elongate member 12 has been illustrated and described as having a bifurcated structural configuration defining an infusion port, inflation port, infusion lumen, inflation lumen, and guide wire lumen, an elongate member can have any suitable structural configuration defining any suitable number of ports and/or lumens. Skilled artisans will be able to select a suitable structural configuration and number of ports and/or lumens for an elongate member according to a particular embodiment based on various considerations, including the structural configuration of a first balloon and/or second balloon of a catheter. For example, alternative to having a bifurcated proximal portion defining an infusion port and an inflation port, as illustrated in Figures 1 and 4, elongate member can comprise a straight, or substantially straight, elongate shaft that has a proximal end defining a first infusion lumen opening providing access to an infusion lumen and/or a first inflation lumen opening providing access to an inflation lumen. Example number of lumens considered suitable for an elongate member include, but are not limited to, one, at least one, a plurality, two, three, four, five, and any other number considered suitable for a particular application. For example, alternative to including a guide wire lumen, an elongate member can omit a guide wire lumen and only define an infusion lumen and an inflation lumen. Optionally, elongate member can define a lumen, such as one in addition to an infusion lumen, inflation lumen, and a guide wire lumen, that has a first opening that is disposed on a proximal portion of the elongate member and a second opening disposed on a distal portion of the elongate member that is adapted to ventilate a bodily passage during the performance of a procedure.

An elongate member can optionally include one or more stiffening elements disposed within the wall, or on a surface, of the elongate member to advantageously increase the rigidity of the elongate member and to facilitate efficient advancement of the distal end of the elongate member towards a point of treatment (e.g., through a stricture). Any suitable stiffening member can be disposed within, on the exterior surface of, or interior surface of, the wall of an elongate member and along the entire length, or a portion of the length, of the elongate member. Skilled artisans will be able to select a suitable stiffening member to include in an elongate member according to a particular embodiment based on various considerations, including the desired rigidity and/or malleability of the elongate member. An example stiffening member considered suitable to include in an elongate member includes, but is not limited to, an elongate rod formed of a flexible and/or malleable material, such as nitinol or stainless steel.

First balloon 14 and second balloon 16 can be formed of any suitable material, and skilled artisans will be able to select a suitable material to form a first balloon and/or a second balloon according to a particular embodiment based on various considerations, including the materials that form an elongate member. Example materials considered suitable to form a balloon include, but are not limited to, biocompatible materials, materials that can be made biocompatible, flexible materials, substantially flexible materials, polymers, Pebax (Pebax is a registered trademark of Ato Chimie Corporation of Allee des Vosges, Courbevoie, France), nylon, polyethylene, and polyurethane. First balloon 14 and second balloon 16 can be formed of the same material or different materials. For example, first balloon 14 can be formed of a first material and second balloon 16 can be formed of a second material that is different than the first material.

In the illustrated embodiment, first balloon 14 is attached to elongate member 12 between elongate member proximal end 18 and elongate member distal end 20 at first balloon proximal junction 44 and first balloon distal junction 46. First balloon 14 comprises a first balloon proximal end 45, a first balloon distal end 47, a first balloon wall 48, a first balloon main body 50, a plurality of protuberances 52, an infusion chamber 54, and a plurality of protuberance pores 56. First balloon 14 is attached to elongate member 12 such that second infusion lumen opening 34 is in communication with infusion chamber 54. With this structural arrangement, first balloon 14 is adapted to move between a deflated configuration and an inflated configuration as fluid and/or medication is moved into and out of infusion chamber 54 via the infusion lumen 26 and second infusion lumen opening 34 and/or when second balloon 16 is moved from a deflated configuration to an inflated configuration, as described in more detail herein. Figures 1, 2, 3, and 4 illustrate first balloon 14 in an inflated configuration.

First balloon proximal junction 44 and first balloon distal junction 46 can comprise any suitable method of attachment between elongate member 12 and first balloon 14, and skilled artisans will be able to select a suitable method of attachment between a balloon and an elongate member according to a particular embodiment based on various considerations, including the materials forming the elongate member and balloon. Example methods of attachment considered suitable between an elongate member and a balloon include, but are not limited to, attachments formed by heat fusing, using adhesives, mechanical connections, and any other method considered suitable for a particular application.

In the illustrated embodiment, each protuberance of the plurality of protuberances 52 has a protuberance length 55, a protuberance height 57, and a protuberance outer surface 58. Each protuberance of the plurality of protuberances 52 extends radially outward from first balloon main body 50 a protuberance height 57 to protuberance outer surface 58 and is positioned along the length of first balloon 14 between first balloon proximal end 45 and first balloon distal end 47. Each protuberance of the plurality of protuberances 52 has a protuberance length 55 that extends along elongate member lengthwise axis 21 from a protuberance proximal end to a protuberance distal end.

Each protuberance of the plurality of protuberances 52 is elongated and positioned along the length of first balloon 14 such that it has a linear, or substantially linear, configuration from a protuberance proximal end to a protuberance distal end. Protuberance outer surface 58 of each protuberance of the plurality of protuberances 52 is disposed radially outward from first balloon main body 50 and is adapted to interact with or damage the wall of a bodily passage (e.g., to cut or penetrate the wall of the bodily passage) when the second balloon 16 is in its inflated configuration, or is moving to its inflated configuration, as described in more detail herein. For example, upon second balloon 16 moving to its inflated configuration, each protuberance of the plurality of protuberances 52 provides a structure for focalizing the dilating pressure of first balloon 14 and/or second balloon 16 at particular locations within a bodily passage. It is considered advantageous to focalize the dilating pressure of first balloon 14 and/or second balloon 16 at least because this provides a mechanism for damaging the wall of a bodily passage in a controlled manner.

Each protuberance of the plurality of protuberances 52 can extend any suitable length along the lengthwise axis of an elongate member and have any suitable height, and skilled artisans will be able to select a suitable length and height for a protuberance according to a particular embodiment based on various considerations, including the desired treatment intended to be performed and/or the amount of damage intended to be accomplished at a point of treatment. Example lengths considered suitable for a protuberance include, but are not limited to, a protuberance that extends from a balloon proximal end to a balloon distal end, a protuberance that extends between a balloon proximal end and a balloon distal end, a protuberance that extends from a balloon proximal end to a location proximal to a balloon distal end, a protuberance that extends beyond a balloon proximal end, a protuberance that extends beyond a balloon distal end, and a protuberance that extends from a location distal to a balloon proximal end to a balloon distal end.

As shown in Figures 2 and 3, a first protuberance 60 of the plurality of protuberances 52 defines a curved cross sectional configuration, a second protuberance 62 of the plurality of protuberances 52 defines a rectangular cross sectional configuration, a third protuberance 64 of the plurality of protuberances 52 defines a multifaceted cross sectional configuration, and a fourth protuberance 66 of the plurality of protuberances 52 defines a pointed cross sectional configuration.

While particular cross sectional configurations have been illustrated and described for the first protuberance 60, second protuberance 62, third protuberance 64, and fourth protuberance 66 of the plurality of protuberances 52, a protuberance can have any suitable cross sectional configuration, and skilled artisans will be able to select a suitable cross sectional configuration for a protuberance according to a particular embodiment based on various configurations, including the desired type of interaction between a protuberance and the wall of a bodily passage. Example cross sectional configurations considered suitable for a protuberance include, but are not limited to, those described herein, rounded, flattened, bead-shaped, asymmetrical, tapered, crown-shaped, pointed, and any other cross sectional configuration considered suitable for a particular application. For example, if greater damage to the wall of a bodily passage is desired, a protuberance having a pointed cross sectional, or a cross sectional configuration that defines a sharp protuberance outer surface, can be used in combination with a balloon. Example protuberance configurations considered suitable to include on a balloon include, but are not limited to, each protuberance of a plurality of protuberances defining the same cross sectional configuration, a first protuberance of a plurality of protuberances defining a first cross sectional configuration and a second protuberance of the plurality of protuberances defining a second cross sectional configuration that is different than the first cross sectional configuration, and each protuberance in a first set of protuberances of a plurality of protuberances defining a first cross sectional configuration and each protuberance in a second set of protuberances of the plurality of protuberances defining a second cross sectional configuration that is different than the first cross sectional configuration. Any suitable number and/or combination of cross sectional configurations can be included on a balloon.

While each protuberance of the plurality of protuberances 52 has been illustrated as integral with the first balloon 14, a protuberance can comprise any suitable material, be integral with the wall of a balloon, or a separate member or feature attached to the wall of a balloon using any suitable method of attachment. Skilled artisans will be able to select a suitable material for a protuberance and a suitable method of attachment to a balloon according to a particular embodiment based on various considerations, including the material(s) that form the balloon. Example materials considered suitable to form a protuberance include, but are not limited to, those described herein, metals, polymers, rigid polymers, polyetheretherketone (PEEK), high-density polyethylene (HDPE), medium-density polyethylene (MDPE), nylon, and any other material considered suitable for a particular application. Example methods of attachment considered suitable between a protuberance and the wall of a balloon include, but are not limited to, forming a protuberance as an integral component of the balloon, using an adhesive, welding, fusing, and any other method of attachment considered suitable for a particular application. When a protuberance is a separate element or feature attached to the wall of a balloon, the protuberance can be formed of a first material and the balloon can be formed of the same first material. Alternatively, when a protuberance is a separate element or feature attached to the wall of a balloon, the protuberance can be formed of a first material and the balloon can be formed of a second material that is different than the first material.

While a plurality of protuberances 52 has been described and illustrated, any suitable number of protuberances can be included in a catheter, and skilled artisans will be able to select a suitable number of protuberances according to a particular embodiment based on various considerations, including the location and size of a treatment site. Example number of protuberances considered suitable to include on a balloon include, but are not limited to, one, at least one, a plurality, two, three, four, five, six, seven, eight, nine, ten, and any other number considered suitable for a particular application.

In the illustrated embodiment, first balloon wall 48, the portion of the exterior surface of elongate member 12 disposed within first balloon 14, and the exterior surface of the second balloon 16 define infusion chamber 54. Infusion chamber 54 is adapted to receive a medication and pass it through each pore of the plurality of protuberance pores 56 such that the medication can be introduced into a bodily passage or the wall of a bodily passage, as described in more detail herein.

In the illustrated embodiment, each protuberance of the plurality of protuberances 52 defines a plurality of protuberance pores 56. Each protuberance pore of the plurality of protuberance pores 56 has the same diameter, a first protuberance opening 68 defined on the interior surface of first balloon 14, and a second protuberance opening 70 defined on protuberance outer surface 58. Thus, each protuberance pore of the plurality of protuberance pores 56 extends through protuberance height 57. Each protuberance pore of the plurality of protuberance pores 56 is in fluid communication with infusion chamber 54 such that fluid introduced into infusion chamber 54 can pass through each protuberance pore of the plurality of protuberances pores 56.

It is considered advantageous to include a plurality of protuberance pores 56 on each protuberance of the plurality protuberances 52 at least because this configuration allows for medication to be passed through each protuberance pore of the plurality of protuberance pores 56 directly into a bodily passage or the wall of a bodily passage when the second balloon 16 is an inflated configuration, or partially inflated configuration, and each protuberance of the plurality of protuberances 52, or a portion thereof, has damaged, or is currently damaging, the wall of the bodily passage. For example, when a bodily passage is being treated with catheter 10, or any other suitable catheter, such as those described herein, positioning a plurality of protuberance pores 56 on each protuberance of the plurality of protuberances 52 provides a mechanism for directing medication being passed through infusion chamber 54 to the portion of the wall of the bodily passage that is being, or has been, damaged by each protuberance of the plurality of protuberances 52.

While a plurality of protuberance pores 56 has been described and illustrated as extending through protuberance height 57 of each protuberance of the plurality of protuberances 52, a pore can extend through any portion of a protuberance and/or a balloon. Skilled artisans will be able to select a suitable portion of a protuberance and/or balloon to define a pore according to a particular embodiment based on various considerations, including the desired amount of medication to be introduced into a bodily passage or the wall of the bodily passage. For example, alternative to a pore extending through protuberance height 57, as shown in Figures 3 and 4, a pore can extend partially through the height of a protuberance such that it defines an opening on the side of the protuberance between the outer surface of the protuberance and the main body of a balloon. Alternatively, a pore can be defined by a balloon wall and extend through the balloon wall and provide access to a chamber defined by the balloon.

While a plurality of protuberance pores 56 has been described and illustrated as defined by each protuberance of the plurality of protuberances 56, a pore can be defined by any suitable feature and/or element of a balloon and at any suitable location on a balloon. Skilled artisans will be able to select a suitable feature and/or element of a balloon to define a pore and/or a suitable location to position a pore according to a particular embodiment based on various considerations, including the desired amount of medication desired to be introduced at a point of treatment. Example arrangements considered suitable to position one or more pores on a balloon include, but are not limited to, defining a wall pore on the main body of a balloon, defining a wall pore on the main body of a balloon and a protuberance pore on a protuberance of a balloon, defining a protuberance pore on a protuberance of a balloon, defining a protuberance pore on a first protuberance of a plurality of protuberances and a second protuberance pore on a second protuberance of the plurality of protuberances, defining a plurality of wall pores on the main body of a balloon, defining a plurality of protuberance pores on a protuberance of a balloon, defining a first set of protuberance pores on each protuberance of a plurality of protuberances and a second, different, set of protuberance pores on the first protuberance of the plurality of protuberances, defining a plurality of wall pores on a main body of a balloon and a protuberance pore on a protuberance of a balloon, defining a plurality of wall pores on a main body of a balloon and a protuberance pore on a first protuberance of a plurality of protuberances and a second protuberance pore on a second protuberance of the plurality of protuberance, and defining a plurality of wall pores on a main body of a balloon and a first set of protuberance pores on a first protuberance of a plurality of protuberances and a second set of protuberance pores on a second protuberance of the plurality of protuberances.

For example, a first set of pores of a plurality of pores can be disposed on a proximal portion of a balloon and a second set of pores of the plurality of pores can be disposed on a distal portion of the balloon such that the distal portion has a greater number of pores than the proximal portion of the balloon. Thus, a greater number of pores can be disposed on a distal portion of a balloon than on a proximal portion of the balloon. Alternatively, a first set of pores of a plurality of pores can be disposed on a proximal portion of a balloon and a second set of pores of the plurality of pores can be disposed on a distal portion of the balloon such that the proximal portion has greater number of pores than distal portion of the balloon. Thus, a greater number of pores can be disposed on a proximal portion of a balloon than on a distal portion of the balloon.

While a plurality of protuberance pores 56 has been illustrated and described as being defined by each protuberance of the plurality of protuberances 52, any suitable number of pores can be provided. Skilled artisans will be able to select a suitable number of pores to include on a balloon according to a particular embodiment based on various considerations, including the desired amount of medication intended to be delivered at a point of treatment. Example number of pores considered suitable to include on a balloon include, but are not limited to, one, at least one, a plurality, two, three, four, five, six, seven, eight, nine, ten, eleven, twelve, thirteen, fourteen, fifteen, and any other number considered suitable for a particular application.

Each pore of the plurality of pores 56 can have any suitable diameter and structural configuration, and skilled artisans will be able to select a suitable diameter and structural configuration for a pore according to a particular embodiment based on various considerations. Example diameters considered suitable for a plurality of pores include, but are not limited to, each pore of a plurality of pores comprising the same diameter, a first pore of the plurality of pores comprising a first diameter and a second pore of the plurality of pores comprising a second diameter that is different than the first diameter, each pore in a first set of pores of a plurality of pores comprising a first diameter and each pore in a second set of pores of the plurality of pores comprising a second diameter that is different than the first diameter. For example, when at least two pores are provided, at least two of the pores can have different diameters. Example structural configurations considered suitable for a pore include, but are not limited to, defining a continuous diameter along the length of a pore, a variable diameter along the length of a pore, a pore that tapers from a first opening to a second opening, a pore that tapers from a second opening to a first opening, a pore that is curved along its length, and any other structural configuration considered suitable for a particular application.

The inventors have determined that pores having a diameter between about .01mm to about .25mm are suitable. In addition, the inventors have determined that pores having a diameter between about .025mm to about .15mm are also suitable. Furthermore, the inventors have determined that pores having a diameter between about .05mm to about .1mm are suitable.

In the illustrated embodiment, second balloon 16 is disposed radially inward of first balloon 14 and is attached to elongate member 12 between elongate member proximal end 18 and elongate member distal end 20 at second balloon proximal junction 72 and second balloon distal junction 74. Second balloon 16 comprises a second balloon wall 76 that defines an inflation chamber 78. Second balloon 16 is attached to elongate member 12 such that second inflation lumen opening 38 is in communication with inflation chamber 78. With this structural arrangement, second balloon 16 is adapted to move between a deflated configuration and an inflated configuration as fluid is moved into and out of inflation chamber 78 via the inflation lumen 28 and second inflation lumen opening 38. Figures 1, 2, 3, and 4 illustrate second balloon 16 in an inflated configuration.

Second balloon proximal junction 72 and second balloon distal junction 74 can comprise any suitable method of attachment between an elongate member 12 and second balloon 16, and skilled artisans will be able to select a suitable method of attachment between a balloon and an elongate member according to a particular embodiment based on various considerations, including the materials forming the elongate member and balloon. Example methods of attachment considered suitable between an elongate member and a balloon include, but are not limited to, attachments formed by heat fusing, using adhesives, mechanical connections, and any other method of attachment considered suitable for a particular application.

While each of the first balloon 14 and second balloon 16 has been described and illustrated as attached to elongate member 12 between elongate member proximal end 18 and elongate member distal end 20, a first balloon and/or second balloon can be positioned at any suitable location along the length of an elongate member. Skilled artisans will be able to select a suitable location to position a balloon on an elongate member according to a particular embodiment based on various considerations, including the structural arrangement of the elongate member. Example locations considered suitable to position a balloon on an elongate member include, but are not limited to, positioning a balloon such that the distal end of the balloon is disposed at the distal end of an elongate member, and positioning a balloon such that the distal end of the balloon is disposed proximal to the distal end of an elongate member.

In use, first balloon 14 is moved between the deflated and inflated configuration by way of movement of second balloon 16 between its deflated configuration and inflated configuration. Thus, a user expands first balloon 14 by inflating second balloon 16 by introducing a fluid, such as saline, into first inflation lumen opening 36, through the inflation lumen 28, second inflation lumen opening 38, and into the inflation chamber 78. The resulting pressure placed on the inner surface of second balloon 16 by the fluid causes first balloon 14 and second balloon 16 to inflate and adopt the inflated configuration. To move first balloon 14 and second balloon 16 to their deflated configurations, vacuum pressure can be applied to inflation lumen 28 (e.g., using a syringe) to remove fluid located within the inflation chamber 78 via second inflation lumen opening 38, inflation lumen 28, and first inflation lumen opening 36 resulting in first balloon 14 and second balloon 16 collapsing and adopting the deflated configurations.

To damage the wall of a bodily passage, such as an airway, sinus passage and/or sinus cavity, a portion, or the entirety, of elongate member distal end 20 and first balloon 14 is disposed in the bodily passage and second balloon 16 is move to its inflated configuration, as described herein. When second balloon 16 is moved to an inflated configuration, depending on the amount of fluid that has been passed into inflation chamber 78, each protuberance of the plurality of protuberances 52, a portion of each protuberance of the plurality of protuberances 52, or a portion of the plurality of protuberances 52, is forced into the wall of the bodily passage to damage the wall of the bodily passage. Thus, a mechanism for damaging the wall of the bodily passage has been described. Subsequently, a medication can be passed through the infusion lumen 26 and into infusion chamber 54 such that the medication can be passed through each protuberance pore of the plurality of protuberance pores 56, or portion of the plurality of protuberance pores 56. This advantageously provides a mechanism for introducing and/or infusing the medication into a bodily passage or the portion of the wall of the bodily passage that is being damaged by each protuberance of the plurality of protuberances 52, a portion of each protuberance of the plurality of protuberances 52, or a portion of the plurality of protuberances 52.

Additional structure can be attached to the catheter 10 to facilitate the inflation and deflation of first balloon 14 and second balloon 16 as described herein. For example, a syringe (not illustrated) or other suitable structure can be attached to the infusion port 22 and/or inflation port 24 using any suitable connection, such as a luer lock connection. A fluid or medication can be stored within the syringe, infusion lumen 26, and/or inflation lumen 28, and can be introduced into and removed from the infusion chamber 54 and/or inflation chamber 78 by operating the syringe using conventional practices.

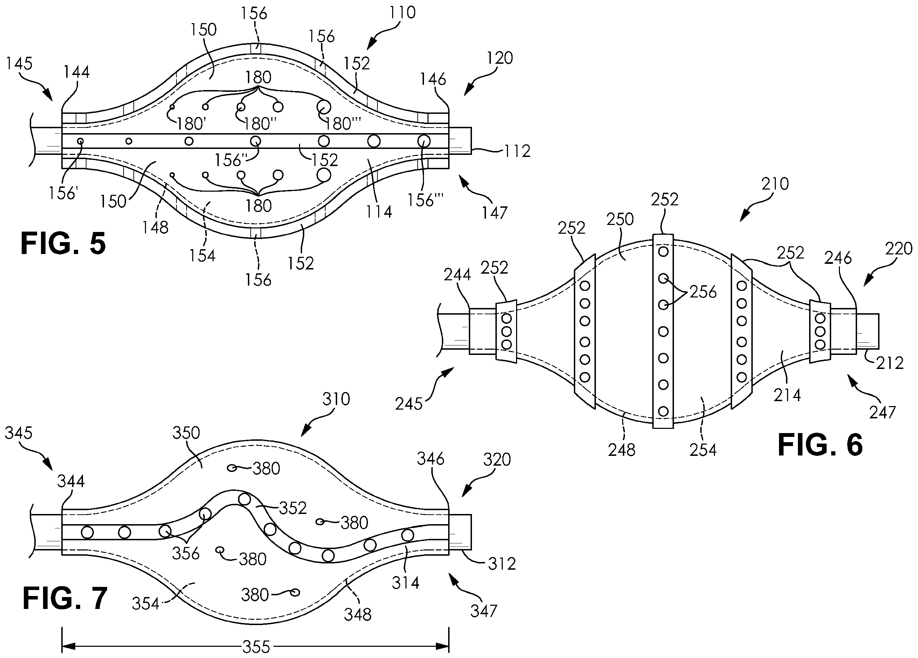

Figure 5 is a side view of the distal end 120 of an alternative catheter 110. Catheter 110 is similar to catheter 10 illustrated in Figures 1, 2, 3, and 4, and described above, except as detailed below. Reference numbers in Figure 5 refer to the same structural element or feature referenced by the same number in Figures 1, 2, 3, and 4, offset by 100. Thus, catheter 110 comprises an elongate member 112, a first balloon 114, and a second balloon (not shown) disposed radially inward of first balloon 114.

In the illustrated embodiment, first balloon 114 comprises a plurality of protuberances 152 and first balloon wall 148 defines a plurality of wall pores 180 between each pair of circumferentially adjacent protuberances of the plurality of protuberances 152. The recitation of a first structural feature "circumferentially adjacent" to a second structural feature means that the first structural feature is the nearest first structural feature to the second structural feature when moving circumferentially along the exterior surface of a balloon.

Each protuberance of the plurality of protuberances 152 defines a plurality of protuberance pores 156 and extends from first balloon proximal end 145 to first balloon distal end 147 and radially outward from main body 150. Thus, alternative to each protuberance of the plurality of protuberances 52 extending only a portion of the length of first balloon 14, as illustrated in Figures 1, 2, 3, and 4, each protuberance of the plurality of protuberances 152 extends the entire length of first balloon 114. Each wall pore of the plurality of wall pores 180 extends through first balloon wall 148 and is in fluid communication with infusion chamber 154 such that fluid introduced into infusion chamber 154 can pass through each wall pore of the plurality of wall pores 180.

In the illustrated embodiment, a first protuberance pore 156' of the plurality of protuberance pores 156 comprises a first diameter and is disposed between first balloon proximal end 145 and first balloon distal end 147. A second protuberance pore 156" of the plurality of protuberance pores 156 comprises a second diameter and is disposed between the first protuberance pore 156' and first balloon distal end 147. A third protuberance pore 156'" of the plurality of protuberance pores 156 comprises a third diameter and is disposed between the second protuberance pore 156" and first balloon distal end 147. The third diameter of the third protuberance pore 156'" is greater than the second diameter of the second protuberance pore 156" and the second diameter of the second protuberance pore 156" is greater than the first diameter of the first protuberance pore 156'.

A first wall pore 180' of the plurality of wall pores 180 comprises a first diameter and is disposed between first balloon proximal end 145 and first balloon distal end 147. A second wall pore 180" of the plurality of wall pores 180 comprises a second diameter and is disposed between the first wall pore 180' and first balloon distal end 147. A third wall pore 180'" of the plurality of wall pores 180 comprises a third diameter and is disposed between the second wall pore 180" and first balloon distal end 147. The third diameter of the third wall pore 180'" is greater than the second diameter of the second wall pore 180" and the second diameter of the second wall pore 180" is greater than the first diameter of the first wall pore 180'.

This configuration is considered advantageous at least because it provides an even distribution, substantially even distribution, or reduced pressure drop, of a medication as it is being passed through infusion chamber 154 and each pore of the plurality of protuberance pores 156 and each pore of the plurality of wall pores 180.

While the plurality of protuberance pores 156 defined by each protuberance of the plurality of protuberances 152 and the plurality of wall pores 180 defined by main body 150 are illustrated as arranged in a linear configuration along the length of first balloon 14, a plurality of pores can be arranged in any suitable configuration on a balloon and/or protuberance. Skilled artisans will be able to select a suitable configuration for a plurality of pores according to a particular embodiment based on various considerations, including the amount of medication desired to be introduced at a treatment site. Example configurations considered suitable for a plurality of pores include, but are not limited to, a plurality of pores that is arranged in a circumferential configuration about the circumference of a balloon, a plurality of pores that is arranged in multiple circumferential configurations about the circumference of a balloon, and a plurality of pores that is arranged in a staggered configuration.

While first balloon wall 148 has been illustrated and described as defining a plurality of wall pores 180 between each pair of circumferentially adjacent protuberances of the plurality of protuberances 152, the wall of a balloon can define any suitable number of wall pores between any suitable pair of circumferentially adjacent protuberances. Skilled artisans will be able to select a suitable number of wall pores and pair of circumferentially adjacent protuberances to dispose one or more wall pores according to a particular embodiment based on various considerations, including the desired amount of medication intended to be introduced into a bodily passage or the wall of a bodily passage. Example number of wall pores considered suitable to include on a balloon include, but are not limited to, one, at least one, a plurality, two, three, four, five, six, seven, eight, nine, ten, and any other number considered suitable for a particular application. Example configurations of wall pores considered suitable to include on a balloon include, but are not limited to, defining one or more wall pores between each pair of circumferentially adjacent protuberances of a plurality of protuberances, defining one or more wall pores between a first protuberance of a plurality of protuberances and a second protuberance of the plurality of protuberances that is circumferentially adjacent to the first protuberance, defining a first set of one or more wall pores between a first pair of circumferentially adjacent protuberances of a plurality of protuberances and defining a second set of one or more wall pores between a second pair of circumferentially adjacent protuberances of the plurality of protuberances.

Figure 6 is a side view of the distal end 220 of a second alternative catheter 210. Catheter 210 is similar to catheter 10 illustrated in Figures 1, 2, 3, and 4, and described above, except as detailed below. Reference numbers in Figure 6 refer to the same structural element or feature referenced by the same number in Figures 1, 2, 3, and 4, offset by 200. Thus, catheter 210 comprises an elongate member 212, a first balloon 214, and a second balloon (not shown) disposed radially inward of first balloon 214.

In the illustrated embodiment, first balloon 214 comprises a plurality of protuberances 252. Each protuberance of the plurality of protuberances 252 defines a plurality of protuberance pores 256 and extends circumferentially about the circumference of first balloon 214 and radially outward from main body 250. Thus, alternative to each protuberance of the plurality of protuberances 52 extending linearly along a portion of the length of first balloon 14, as illustrated and described with respect to Figures 1, 2, 3, and 4, each protuberance of the plurality of protuberances 252 extends about the entire circumference of first balloon 214.

While each protuberance of the plurality of protuberances 252 has been illustrated and described as extending about the entire circumference of first balloon 214, a protuberance can extend about any suitable length of the circumference of a balloon. Skilled artisans will be able to select a suitable length to define a protuberance along the circumference of a balloon according to a particular embodiment based on various considerations, including the desired treatment intended to be performed. Example lengths considered suitable to define a protuberance include, but are not limited to, defining a protuberance about the entire circumference of a balloon, and defining a protuberance about a portion of the circumference of a balloon.

In the illustrated embodiment, no pores are defined on main body 250 of first balloon 214. This configuration is considered advantageous at least because when catheter 210 is disposed in a bodily passage and the second balloon is in the inflated configuration, thus placing the first balloon 214 in the inflated configuration, medication being introduced into infusion chamber 254 is directed through each protuberance pore of the plurality of protuberance pores 256 and each protuberance of the plurality of protuberances 252. As a result, the medication being passed through each protuberance pore of the plurality of protuberance pores 256 is directed to the portion of the bodily passage in contact with or interacting with each protuberance of the plurality of protuberances 252 (e.g., the portion of the wall of the bodily passage being damaged by each protuberance of the plurality of protuberances 252).

Alternative to, or in combination with, defining a plurality of protuberance pores 256 on each protuberance of the plurality of protuberances 252, as shown in Figure 6, one or more pores can be defined on each protuberance of the plurality of protuberances and/or by main body of a balloon.

Figure 7 is a side view of the distal end 320 of a third alternative catheter 310. Catheter 310 is similar to catheter 10 illustrated in Figures 1, 2, 3, and 4, and described above, except as detailed below. Reference numbers in Figure 7 refer to the same structural element or feature referenced by the same number in Figures 1, 2, 3, and 4, offset by 300. Thus, catheter 310 comprises an elongate member 312, a first balloon 314, and a second balloon (not shown) disposed radially inward of first balloon 314.

In the illustrated embodiment, first balloon 314 comprises a protuberance 352 and a plurality of wall pores 380. Protuberance 352 defines a plurality of protuberance pores 356 and extends from first balloon proximal end 345 to first balloon distal end 347 and radially outward from main body 350. Thus, alternative to each protuberance of the plurality of protuberances 52 extending along only a portion of the length of first balloon 14, as illustrated in Figures 1, 2, 3, and 4, first balloon 314 defines a single protuberance 352 that extends the along the entire length of first balloon 314. Protuberance 352 is a curved member extending along protuberance length 355; i.e., it is non-linear, or substantially non-linear. Each wall pore of the plurality of wall pores 380 extends through first balloon wall 348 and is in fluid communication with infusion chamber 354 such that fluid introduced into infusion chamber 354 can pass through each wall pore of the plurality of wall pores 380.

In the illustrated embodiment, each pore of the plurality of protuberance pores 356 has a first diameter and each pore of the plurality of wall pores 380 has a second diameter that is less than the first diameter. This configuration is considered advantageous at least because it provides a mechanism for compensating for the increased pressure that protuberance 352 will experience in use because it extends radially outward from main body 350 and is forced into the wall of a bodily passage, which may contain harder tissue (e.g., scar tissue, lesions). Thus, in use, the increased resistance provided by the wall of the bodily passage on protuberance 352 would be compensated by decreased resistance in each protuberance pore of the plurality of protuberance pores 356 because each protuberance pore of the plurality of protuberance pores 356 has a larger diameter than each wall pore of the plurality of wall pores 380.

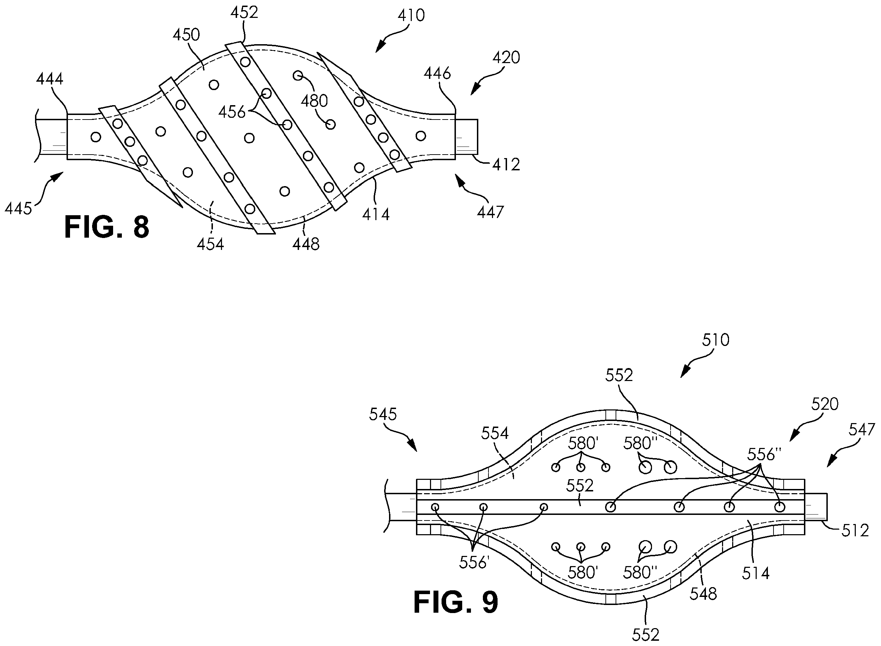

Figure 8 is a side view of the distal end 420 of a fourth alternative catheter 410. Catheter 410 is similar to catheter 10 illustrated in Figures 1, 2, 3, and 4, and described above, except as detailed below. Reference numbers in Figure 8 refer to the same structural element or feature referenced by the same number in Figures 1, 2, 3, and 4, offset by 400. Thus, catheter 410 comprises an elongate member 412, a first balloon 414, and a second balloon (not shown) disposed radially inward of first balloon 414.

In the illustrated embodiment, first balloon 414 comprises a protuberance 452 and a plurality of wall pores 480. Protuberance 452 extends from first balloon proximal end 445 to first balloon distal end 447, radially outward from main body 450, and about the circumference of first balloon 414 multiple times. Thus, alternative to including a plurality of protuberances 52 that extend only a portion of the length of first balloon 14, as illustrated in Figures 1, 2, 3, and 4, protuberance 452 extends the entire length of first balloon 414 and about the circumference of first balloon 414 multiple times. Each wall pore of the plurality of wall pores 480 extends through first balloon wall 448 and is in fluid communication with infusion chamber 454 such that fluid introduced into infusion chamber 454 can pass through each wall pore of the plurality of wall pores 480.

While a single protuberance 452 has been illustrated and described as extending the entire length of first balloon 414 and about the circumference of first balloon 414 multiple times, any suitable number of protuberances can extend along any suitable length of a balloon and about the circumference of the balloon any suitable number of times. Skilled artisans will be able to select a suitable structural configuration for any suitable number of protuberances according to a particular embodiment based on various considerations, including the desired treatment intended to be completed. For example, alternative to including a single protuberance that extends about the circumference of a balloon multiple times and along the entire length of the balloon, a plurality of protuberances can be included in a balloon that extend about the entire circumference of the balloon, or a portion of the circumference of the balloon, and the entire length, or a portion of the length, of the balloon.

Figure 9 is a side view of the distal end 520 of a fifth alternative catheter 510. Catheter 510 is similar to catheter 110 illustrated in Figure 5, and described above, except as detailed below. Reference numbers in Figure 9 refer to the same structural element or feature referenced by the same number in Figure 5, offset by 400. Thus, catheter 510 comprises an elongate member 512, a first balloon 514, and a second balloon (not shown) disposed radially inward of first balloon 514.

In the illustrated embodiment, each protuberance of the plurality of protuberances 552 defines a first set of protuberance pores 556' of the plurality of protuberance pores 556 and a second set of protuberance pores 556" of the plurality of protuberance pores 556. Each protuberance pore in the first set of protuberance pores 556' comprises a first diameter and is disposed between first balloon proximal end 545 and first balloon distal end 547. Each protuberance pore in the second set of protuberance pores 556" comprises a second diameter and is disposed between the first set of protuberance pores 556' and first balloon distal end 547. The diameter of each protuberance pore in the second set of protuberance pores 556" is greater than the diameter of each protuberance pore in the first set or protuberance pores 556'.

In the illustrated embodiment, first balloon wall 548 defines a first set of wall pores 580' of the plurality of wall pores 580 and a second set of wall pores 580" of the plurality of wall pores 580. Each wall pore in the first set of wall pores 580' comprises a first diameter and is disposed between first balloon proximal end 545 and first balloon distal end 547. Each wall pore in the second set of wall pores 580" comprises a second diameter and is disposed between the first set of wall pores 580' and first balloon distal end 547. The diameter of each wall pore in the second set of wall pores 580" is greater than the diameter of each wall pore in the first set of wall pores 580'.

This configuration is considered advantageous at least because it provides an even distribution, substantially even distribution, or reduced pressure drop, of a medication as it is being passed through the infusion chamber 554 and each pore of the plurality of protuberance pores 556 and each pore of the plurality of wall pores 580.

While the diameter of each pore in the second set of protuberance pores 556" has been described and illustrated as greater than the diameter of each pore in the first set of protuberance pores 556' and the diameter of each pore in the second set of wall pores 580" has been described and illustrated as greater than the diameter of each pore in the first set of wall pores 580', other configurations are considered suitable. Skilled artisans will be able to select a suitable configuration for a first set of pores and a second set of pores of according to a particular embodiment based on various considerations, including the amount of medication desired to be introduced into a bodily passage. For example, each protuberance pore in a first set of protuberance pores of a plurality of protuberance pores, disposed between a first balloon proximal end and first balloon distal end, can have a first diameter that is greater than each protuberance pore in a second set of protuberance pores of the plurality of protuberance pores that is disposed between the first set of protuberance pores and the first balloon distal end. In a second example, and in combination with or alternative to the above example, each wall pore in a first set of wall pores of a plurality of wall pores, disposed between a first balloon proximal end and first balloon distal end, can have a first diameter that is greater than each wall pore in a second set of wall pores of the plurality of wall pores that is disposed between the first set of wall pores and the first balloon distal end. Alternatively, the diameter of each protuberance pore in a first set of protuberance pores and/or a second set of protuberance pores of a plurality of protuberance pores can be different than the diameter of each pore in a first set of wall pores and/or the second set of wall pores of a plurality of wall pores.

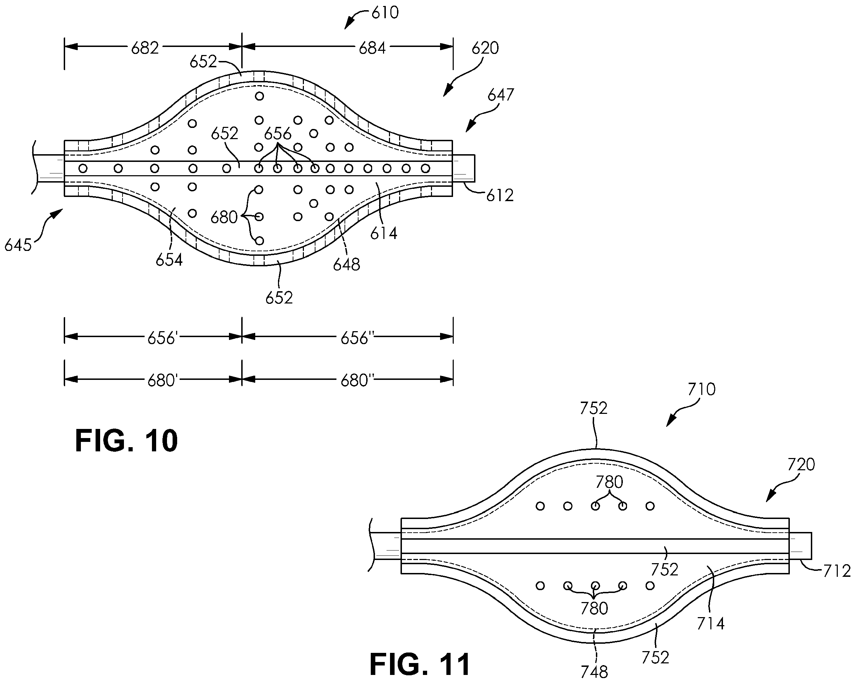

Figure 10 is a side view of the distal end 620 of a sixth alternative catheter 610. Catheter 610 is similar to catheter 110 illustrated in Figure 5, and described above, except as detailed below. Reference numbers in Figure 10 refer to the same structural element or feature referenced by the same number in Figure 5, offset by 500. Thus, catheter 610 comprises an elongate member 612, a first balloon 614, and a second balloon (not shown) disposed radially inward of first balloon 614.

In the illustrated embodiment, first balloon 614 has a first balloon proximal portion 682 and first balloon distal portion 684, each protuberance of the plurality of protuberances 652 defines a plurality of protuberance pores 656, and first balloon wall 648 defines a plurality of wall pores 680. Each protuberance of the plurality of protuberances 652 has a first set of protuberance pores 656' of the plurality of protuberance pores 656 and a second set of protuberance pores 656" of the plurality of protuberance pores 656. Each protuberance pore in the first set of protuberance pores 656' is defined on first balloon proximal portion 682 and each protuberance pore in the second set of protuberance pores 656" is defined on the first balloon distal portion 684. Each protuberance pore in the first set of protuberance pores 656' and second set of protuberance pores 656" comprises a first diameter and is disposed between first balloon proximal end 645 and first balloon distal end 647.

In the illustrated embodiment, first balloon wall 648 defines a first set of wall pores 680' of the plurality of wall pores 680 and a second set of wall pores 680" of the plurality of wall pores 680. Each wall pore in the first set of wall pores 680' is defined on first balloon proximal portion 682 and each wall pore of the second set of wall pores 680" is defined on first balloon distal portion 684. Each wall pore in the first set of wall pores 680' and second set wall pores 680" comprises a first diameter and is disposed between first balloon proximal end 645 and first balloon distal end 647. The diameter of each wall pore in the first set of wall pores 680' and second set of wall pores 680" is equal to, or substantially equal to, the diameter of each protuberance pore in the first set of protuberance pores 656' and second set of protuberance pores 656".

This configuration is considered advantageous at least because it provides an even distribution, substantially even distribution, or reduced pressure drop, of a medication as it is being passed through the infusion chamber 654 and each pore of the plurality of protuberance pores 656 and each pore of the plurality of wall pores 680.

While the diameter of each pore in the first set of protuberance pores 656' and second set of protuberance pores 656" has been described and illustrated as equal to, or substantially equal to, the diameter of each pore in the first set of wall pores 680' and second set of wall pores 680", any suitable diameter can be used for a protuberance pore and/or wall pore of a balloon. Skilled artisans will be able to select a suitable diameter for a wall pore and/or protuberance pore of a balloon according to a particular embodiment based on various considerations, including the amount of medication being introduced into a bodily passage or the wall of a bodily passage. Example diameters considered suitable for a plurality of wall pores and/or protuberance pores include, but are not limited to, each protuberance pore in a first set of protuberance pores of a plurality of protuberance pores and a second set of protuberance pores of the plurality of protuberance pores having a first diameter and each wall pore in a first set of wall pores of a plurality of wall pores and a second set of wall pores of the plurality of wall pores having a second diameter that is different than, greater than, and/or less than, the first diameter.

Figure 11 is a side view of the distal end 720 of a seventh alternative catheter 710. Catheter 710 is similar to catheter 110 illustrated in Figure 5, and described above, except as detailed below. Reference numbers in Figure 11 refer to the same structural element or feature referenced by the same number in Figure 5, offset by 600. Thus, catheter 710 comprises an elongate member 712, a first balloon 714, and a second balloon (not shown) disposed radially inward of first balloon 714.

In the illustrated embodiment, each protuberance of the plurality of protuberances 752 omits the inclusion of a plurality of pores and first balloon wall 748 defines a plurality of wall pores 780. Each wall pore of the plurality of wall pores 780 comprises a first diameter. Thus, each wall pore of the plurality of wall pores 780 has the same diameter.

This configuration is considered advantageous at least because it provides a mechanism for introducing a medication to a portion of the wall of a bodily passage that is not being damaged by a protuberance of the plurality of protuberances.

While each wall pore of the plurality of wall pores 780 has been described and illustrated as having the same diameter, a wall pore can comprise any suitable diameter, and skilled artisans will be able to select a suitable diameter for a pore according to a particular embodiment based on various considerations, including the desired amount of medication to be introduced into a bodily passage. For example, a first wall pore can comprise a first diameter and a second wall pore disposed distal to the first wall pore can comprise a second diameter that is greater than the first wall pore and third wall pore disposed distal to the second wall pore can comprise a third diameter that is greater than the second wall pore.

Any combination of the herein described catheter and/or balloon configurations, features, elements, protuberances, protuberance pores, wall pores, and/or structural arrangements is considered suitable, and skilled artisans will be able to select a suitable configuration, feature, element, protuberance, protuberance pore, wall pore, and/or structural arrangement for a catheter and/or balloon according to a particular embodiment based on various considerations.

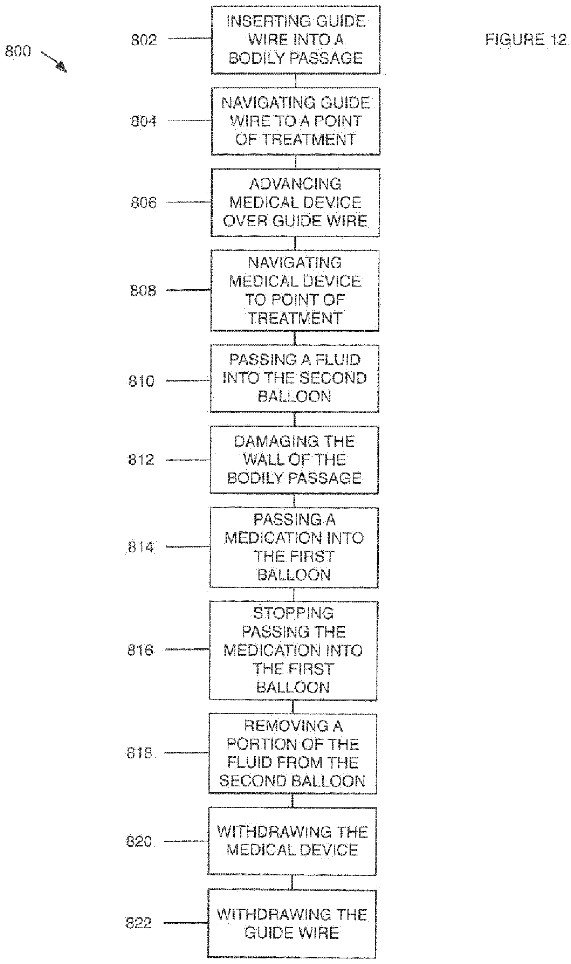

Figure 12 is a flowchart representation of an exemplary method 800 of treating a bodily passage.

An initial step 802 comprises inserting a guide wire having a guide wire proximal end and a guide wire distal end into a bodily passage such that the guide wire distal end is disposed within the bodily passage. Another step 804 comprises navigating the guide wire distal end to a point of treatment within the bodily passage. Another step 806 comprises advancing a medical device (e.g., catheter 10, catheter 110, catheter 210, catheter 310, catheter 410, catheter 510, catheter 610, catheter 710) having a medical device proximal end and a medical device distal end over the previously placed guide wire such that the medical device distal end is disposed within the bodily passage. Another step 808 comprises navigating the medical device distal end to a point of treatment within the bodily passage. Another step 810 comprises passing a fluid (e.g., saline) through an inflation lumen and into an inflation chamber of a second balloon with a pressure sufficient to inflate the second balloon and provide contact between a portion, the entirety, or a majority, of a first balloon and the wall of the bodily passage. Another step 812 comprises damaging the wall of the bodily passage. Another step 814 comprises passing a medication through an infusion lumen into an infusion chamber of the first balloon with a pressure sufficient to expel the medication through each pore of a plurality of pores, or a portion of the plurality of pores. Another step 816 comprises stopping the step of passing a medication through the infusion lumen and into the first balloon. Another step 818 comprises removing a portion, or the entirety, of the fluid from the inflation chamber of the second balloon. Another step 820 comprises withdrawing the medical device distal end from the bodily passage. Another step 822 comprises withdrawing the guide wire distal end from the bodily passage.

The step 802 of inserting a guide wire having a guide wire proximal end and a guide wire distal end into a bodily passage such that the guide wire distal end is disposed in the bodily passage can be accomplished using a guide wire independent of any other device. Alternatively, a guide wire can pass through the working channel of a visualization scope. Any suitable guide wire having any suitable length and formed of any suitable material can be used to accomplish this step. Optionally the guide wire can comprise a guide wire distal end having a light source and/or a camera disposed thereon and configured to emit light from the guide wire distal end and/or configured to transmit images to a display. It is considered advantageous to include a light source and/or camera to assist in positioning the guide wire distal end at a point of treatment.

Optionally, the step 802 of inserting a guide wire having a guide wire proximal end and a guide wire distal end into a bodily passage such that the guide wire distal end is disposed in the bodily passage can be omitted from the herein described methodologies and an optional step can comprise inserting a medical device that is preloaded over a guide wire into a bodily passage such that the medical device distal end is disposed in the bodily passage. Thus, a medical device can be preloaded over a guide wire and the combined medical device and guide wire can be introduced into a bodily passage in combination with one another.

The step 804 of navigating the guide wire distal end to a point of treatment within the bodily passage can be accomplished using direct visualization (e.g., with a scope). Alternatively, when the guide wire distal end comprises a light source and/or imaging device (e.g., camera), navigating the guide wire distal end to a point of treatment can be accomplished transcutaneously and/or via live feed on a display. For example, when the guide wire distal end comprises a light source, navigating the guide wire distal end to a point of treatment can be accomplished using transcutaneous visualization by visualizing the intensity of the light to determine if the intensity is indicative of proper positioning of the guide wire distal end at a point of treatment. When the guide wire distal end comprises an imaging device, navigating the guide wire distal end to a point of treatment can be accomplished by viewing images provided on a display and determining if the images indicate proper positioning of the guide wire distal end.

The step 806 of advancing a medical device having a medical device proximal end and a medical device distal end over the previously placed guide wire such that the medical device distal end is disposed within the bodily passage can be accomplished using any suitable medical device. Skilled artisans will be able to select a suitable medical device according to a particular embodiment based on various considerations, including the location of the point of treatment and the type of treatment intended to be performed. Example medical devices considered suitable include, but are not limited to, the catheters described herein, such as catheter 10, catheter 110, catheter 210, catheter 310, catheter 410, catheter 510, catheter 610, catheter 710, variations thereof, and any other catheter considered suitable for a particular application. An exemplary catheter that can be used to accomplish the methodologies, steps, alternative steps, and/or optional steps described herein is illustrated and described with respect to Figures 1, 2, 3, and 4, and comprises an elongate member 12, a first balloon 14, and a second balloon 16.

The step 806 of advancing a medical device distal end over the previously placed guide wire such that the medical device distal end is disposed in the bodily passage can be accomplished by inserting the guide wire proximal end through guide wire lumen 30 defined by elongate member 12 and advancing the catheter 10 distally over the previously placed guide wire 43. Alternatively, the steps of inserting the guide wire distal end into a bodily passage and navigating the guide wire distal end to a point of treatment can be omitted and the medical device distal end can be inserted into the bodily passage independently or through the working channel of a scope, or along-side a scope.

While the steps of navigating the guide wire distal end to a point of treatment within the bodily passage and advancing a medical device over the previously placed guide wire such that the medical device distal end is disposed in the bodily passage have been described as separate steps, these steps can be accomplished concurrently. Alternatively, the step of advancing a medical device over the previously placed guide wire such that the medical device distal end is disposed in the bodily passage can be accomplished prior to the step of navigating the guide wire distal end to a point of treatment within the bodily passage.

The step 808 of navigating the medical device distal end to a point of treatment within the bodily passage can be accomplished using direct visualization of the medical device distal end (e.g., with a scope). Optionally, the medical device distal can include a light source configured to emit light from the medical device and/or an imaging device (e.g., camera) configured to transmit images to a display. It is considered advantageous to include a light source and/or an imaging device on a medical device to assist with positioning the medical device distal end at a point of treatment. When the medical device distal end includes a light source and/or an imaging device, navigating the medical device distal end to a point of treatment can be accomplished transcutaneously and/or via live feed on a display, as described herein.

While the steps of navigating the guide wire distal end to a point of treatment within the bodily passage and navigating the medical device distal end to a point of treatment within the bodily passage have been described as separate steps, these steps can be accomplished concurrently. In addition, while navigating the guide wire distal end to a point of treatment has been described, the guide wire distal end can alternatively be navigated proximal to, or distal to, the point of treatment. Furthermore, while navigating the medical device distal end to a point of treatment has been described, the medical device distal end can alternatively be navigated proximal to, or distal to, the point of treatment.

Alternatively, the steps of advancing the medical device distal end into a bodily passage and navigating the medical device distal end to a point of treatment can be accomplished using a guiding catheter, with or without the use of a guide wire. When a guiding catheter is used in combination with a guide wire, an initial step comprises inserting the guiding catheter distal end into a bodily passage. Another step comprises navigating the guiding catheter distal end to a point of treatment using any suitable visualization technique, such as those described herein. Another step comprises inserting the guide wire distal end into a lumen of the guiding catheter. Another step comprises navigating the guide wire distal end to a point of treatment. Another step comprises advancing the medical device distal end over the guide wire. Another step comprises navigating the medical device distal end over the guide wire to a point of treatment.

The guiding catheter can optionally include a light source configured to emit light from the guiding catheter and/or an imaging device (e.g., camera) configured to transmit images to a display. It is considered advantageous to include a light source and/or an imaging device on a guiding catheter to assist in positioning the distal end of the guiding catheter at a point of treatment. When the guiding catheter distal end includes a light source and/or an imaging device, navigating the guiding catheter to a point of treatment can be accomplished transcutaneously and/or via live feed on a display, as described herein.

Alternatively, the steps of advancing the medical device distal end into a bodily passage and navigating the medical device distal end to a desired point of treatment can be accomplished using a guiding catheter independent of a guide wire. When a guiding catheter is used independent of a guide wire, an initial step comprises inserting the guiding catheter distal end into a bodily passage. Another step comprises navigating the guiding catheter distal end to a point of treatment using any suitable visualization technique, such as those described herein. Another step comprises inserting a medical device distal end through a lumen of the guiding catheter. Another step comprises navigating the medical device distal end through the lumen of the guiding catheter to a point of treatment.

An optional step that can be completed concurrently with, or subsequent to, the step of navigating the medical device distal end to a point of treatment within said bodily passage comprises confirming placement of each protuberance of the plurality of protuberances such that each protuberance of the plurality of protuberances, a portion of each protuberance of the plurality of protuberances, a portion of the plurality of protuberances, or a majority of the plurality of protuberances, is disposed adjacent to, or substantially adjacent to, a previously selected point of treatment. This optional step is considered advantageous at least because it focalizes the dilating pressure of each protuberance of the plurality of protuberances, a portion of each protuberance of the plurality of protuberances, a portion of the plurality of protuberances, or a majority of the plurality of protuberances, such that the wall of a bodily passage can be damaged in a controlled manner.

The step 810 of passing a fluid through an inflation lumen and into an inflation chamber with a pressure sufficient to inflate the second balloon and provide contact between a portion, the entirety, or a majority, of a first balloon and the wall of the bodily passage can be accomplished by introducing a fluid into the inflation chamber 78 to advance second balloon 16 from its deflated configuration to an inflated configuration. The amount of the exterior surface of first balloon 14 and/or each protuberance of the plurality of protuberances 52 that contacts the wall of the bodily passage, and the amount of pressure exerted by the exterior surface of first balloon 14 and/or each protuberance of the plurality of protuberances 52 onto the wall of the bodily passage, will depend on the amount fluid being passed into inflation chamber 78.

For example, in procedures where a large amount of contact and pressure are desired between the exterior surface of first balloon 14 and/or each protuberance of the plurality of protuberances 52, a greater amount of fluid will be required to be introduced into inflation chamber 78 as compared to procedures in which a lower amount of contact and pressure are desired between the exterior surface of first balloon 14 and/or each protuberance of the plurality of protuberances 52 and the wall of the bodily passage. The inventors have determined that pressures in the range from about 1 ATM to about 20 ATM are considered suitable to inflate a second balloon 16 and provide contact and pressure between the exterior surface of first balloon 14 and/or each protuberance of the plurality of protuberances 52. The amount of pressure required to inflate a balloon and provide contact and pressure with a surface will be based on various factors including, but not limited to, the material forming the balloon, the type of surface in which the balloon is contacting, and the fluid being introduced into the inflation chamber of the balloon. For example, balloons with smaller inflated diameters may require higher pressures than balloons with larger inflated diameters to produce a desired amount of contact and pressure between the balloon and a surface.

Example fluids considered suitable to introduce into an inflation chamber 78 to advance second balloon 16 to an inflated configuration include, but are not limited to, saline, water, contrast, or a mixture of one or more of saline, water, and/or contrast.

The step 812 of damaging the wall of the bodily passage can be accomplished by continuing to pass fluid into the inflation chamber 78 to further inflate second balloon 16. The amount of damage caused by the exterior surface of first balloon 14 and/or each protuberance of the plurality of protuberances 52 contacting the wall of the bodily passage will depend on the amount fluid being passed, or introduced, into inflation chamber 78.

For example, in procedures where a large amount of damage is desired to be produced in the wall of a bodily passage by the exterior surface of first balloon 14 and/or each protuberance of the plurality of protuberances 52, a greater amount of fluid will be required to be introduced into inflation chamber 78 as compared to procedures in which a lesser amount of damage is desired to be produced in the wall of the bodily passage by the exterior surface of first balloon 14 and/or each protuberance of the plurality of protuberances 52. The inventors have determined that pressures in the range from about 1 ATM to about 20 ATM are considered suitable to inflate second balloon 16 to produce damage in the wall of a bodily passage.

As second balloon 16 is advanced to the inflated configuration and fluid is introduced into the inflation chamber 78, each protuberance of the plurality of protuberances 52 is adapted to damage the wall of the bodily passage (e.g., by being forced into the wall of the bodily passage). The amount of damage that will occur in the wall of the bodily passage will depend on the amount of fluid introduced into inflation chamber 78. It is considered advantageous to damage the wall of a bodily passage at least because this allows for proper dilation of the bodily passage, especially in instances where the bodily passage includes build up, and provides a mechanism for introducing a medication directly into the wall of the bodily passage. For example, damaging the wall of a bodily passage provides one or more areas in the wall of the bodily passage that allow for the infusion of medication being passed through each pore of the plurality of pores 56, or a portion of the plurality of pores 56, directly into the wall of the bodily passage, as described herein.

The step 812 of damaging the wall of the bodily passage can be performed concurrently, or after, the step of passing a fluid through the inflation lumen and into the second balloon with a pressure to inflate the second balloon and provide contact between a portion, the entirety, or the majority, of the first balloon and the wall of the bodily passage.

The step 814 of passing a medication through an infusion lumen into an infusion chamber of the first balloon with a pressure sufficient to expel the medication through each pore of the plurality of pores, or a portion of the plurality of pores, can be accomplished by passing a medication through infusion port 22 and infusion lumen 26. The medication can be passed into infusion lumen 26 using any suitable device (e.g., a syringe in communication with the infusion lumen). By inflating second balloon 16 to a pressure suitable to damage the wall of the bodily passage and provide interaction between the exterior surface of first balloon 14 and/or each protuberance of the plurality of protuberances 52 and the wall of the bodily passage, the medication being expelled by the each pore of the plurality of pores 56, or portion of the plurality of pores 56, can advantageously be infused into the wall of the bodily passage. As described herein, each pore of the plurality of pores 56 permits medication to pass through the pore, preferably with the application of pressure within the infusion chamber 54. Depending on the size and number of pores defined on first balloon 14, the medication can be introduced into a bodily passage, or infused into the wall of a bodily passage, at a variety of rates.

For example, in procedures where it is desired to infuse a larger amount of medication at a point of treatment, a larger amount of fluid will be passed through infusion port 24 into infusion chamber 54 as compared to procedures where it is desired to infuse a lower amount of medication at a point of treatment. The inventors have determined that pressures in the range from about 1 ATM to about 10 ATM are considered suitable to expel the medication through each pore of the plurality of pores 56, or portion of the plurality of pores 56, and infuse the medication into the wall of the bodily passage. Skilled artisans will appreciate however, that the pressure required to infuse the medication will correlate with the inflated pressure of the second balloon.

Any suitable medication can be used in accordance with the embodiments described herein, and skilled artisans will be able to select a suitable medication according to a particular embodiment based on various considerations, including the desired treatment intended to be performed. Example medications considered suitable to infuse in a bodily passage, or into the wall of a bodily passage, include, but are not limited to, anti-inflammatories (e.g., steroids), antineoplastics (e.g., mitomycin, mitomycin C), cytotoxics, adrenaline (e.g., epinephrine), antibiotics, antifungal agents, anti-proliferatives (e.g., such as those used on coronary stents), therapeutic agents, stem cells, a combination of any of the described medications, and any other medication considered suitable for a particular application.

Step 814 can be accomplished prior to, concurrent with, or subsequent to the steps of navigating the medical device distal end to a point of treatment within said bodily passage, and/or passing a fluid through an inflation lumen and into an inflation chamber. Alternatively, the step of navigating the medical device distal end to a point of treatment within said bodily passage can be accomplished concurrently with the step of passing a fluid through an inflation lumen and into the second balloon with a pressure sufficient to inflate the second balloon and provide contact between a portion of the first balloon and the passage wall. Alternatively, the step of navigating the medical device distal end to a point of treatment within said bodily passage can be accomplished concurrently with the step of passing a medication through the infusion lumen into the first balloon with a pressure sufficient to expel the medication through each pore of the plurality of pores, or a portion of the plurality of pores. Alternatively, the step of passing a medication through the infusion lumen into the first balloon with a pressure sufficient to expel the medication through each pore of the plurality of pores, or portion of the plurality of pores, can be accomplished prior to or concurrently with the step of passing a fluid through the inflation lumen and into the second balloon with a pressure sufficient to inflate the second balloon and provide contact between a portion of the first balloon and the passage wall and/or the step of navigating the medical device distal end to a point of treatment within said bodily passage.

The step 816 of stopping the step of passing a medication through the infusion lumen and into the first balloon can be accomplished by terminating the passing of the medication through infusion lumen 26 and into the infusion chamber 54. When a syringe is being used to pass medication into the infusion lumen, this can be accomplished by removing pressure from the plunger of the syringe.

The step 818 of removing a portion, or the entirety, of the fluid from the inflation chamber of the second balloon can be accomplished by removing a portion, or the entirety, of the fluid passed into the inflation chamber 78. For example, a syringe in communication with first inflation lumen opening 36 can be used to provide vacuum pressure to remove the fluid from the inflation chamber 78. The amount of fluid removed from inflation chamber 78 can vary depending on the procedure. For example, alternative to removing a portion of the fluid, all of the fluid, or as much as possible, can be removed from inflation chamber 78.

While the steps of stopping the step of passing a medication through the infusion lumen and into the first balloon and removing a portion of the fluid from the second balloon have been described as separate steps, these steps can be accomplished concurrently. Alternatively, the step of stopping the step of passing a medication through the infusion lumen and into the first balloon can be accomplished prior to the step of removing a portion of the fluid from the second balloon. Alternatively, the step of removing a portion, or the entirety, of the fluid from the inflation chamber of the second balloon can be accomplished between the step of damaging the wall of the bodily passage (e.g., step 812) and the step of passing a medication through an infusion lumen into an infusion chamber of the first balloon with a pressure sufficient to expel the medication through each pore of the plurality of pores, or a portion of the plurality of pores (e.g., step 814). Performing the steps in this order is considered advantageous at least because it reduces the overall pressure in second balloon and allows medication to exit the plurality of pores, or a portion of the plurality of pores, with less resistance as compared to when the second balloon is in a more inflated configuration.

The step 820 of withdrawing the medical device distal end from the bodily passage can be accomplished by pulling the medical device proximally over the guide wire 43 until it is completely removed from the bodily passage. Alternatively, if the medical device distal end has been navigated to a point of treatment independent of a guide wire, the step of withdrawing the medical device distal end from the bodily passage can be accomplished by pulling the medical device proximally until the medical device distal end is completely removed from the bodily passage.

While the steps of passing a medication through the infusion lumen into the first balloon with a pressure sufficient to expel the medication through each pore of the plurality of pores, or a portion of the plurality of pores, removing a portion of the fluid from the second balloon, and withdrawing the medical device distal end from the bodily passage have been described as separate steps, these steps can be accomplished concurrently. Alternatively, the steps of passing a medication through the infusion lumen into the first balloon with a pressure sufficient to expel the medication through each pore of the plurality of pores, or a portion of the plurality of pores, and removing a portion of the fluid from the second balloon can be accomplished concurrently and the step of withdrawing the medical device distal end can subsequently be accomplished. Alternatively, the step of passing a medication through the infusion lumen into the first balloon with a pressure sufficient to expel the medication through each pore of the plurality of pores, or a portion of the plurality, can be accomplished and the steps of removing a portion of the fluid from the second balloon and withdrawing the medical device distal end from the bodily passage can be accomplished subsequently and concurrently.

While the steps of stopping the step of passing a medication through the infusion lumen and into the first balloon, removing a portion of the fluid from the second balloon, and withdrawing the medical device distal end from the bodily passage have been described as separate steps, these steps can be accomplished concurrently. Alternatively, the step of stopping the step of passing a medication through the infusion lumen and into the first balloon can be accomplished and the steps removing a portion of the fluid from the second balloon and withdrawing the medical device distal end from the bodily passage can be accomplished concurrently.

The step 822 of withdrawing the guide wire distal end from the bodily passage can be accomplished by pulling the guide wire proximally until it is completely removed from the bodily passage. Alternatively, this step can be accomplished in combination with withdrawing the medical device from the bodily passage.

If a guiding catheter has been used to advance the medical device to a point of treatment, a required additional step of withdrawing the guiding catheter distal end from the bodily passage is accomplished concurrently with withdrawing the medical device distal end and/or guide wire, or subsequent to withdrawing the medical device distal end and/or guide wire.

Optional steps of inserting the distal end of a cutting tool into the bodily passage, navigating the distal end of the cutting tool to a point of treatment, cutting tissue from the bodily passage, removing the tissue, and withdrawing the cutting tool can be accomplished prior to advancing the medical device, guiding catheter, and/or guide wire into a bodily passage. Any suitable cutting tool can be used to accomplish these steps. For example, the cutting tool can comprise a suction cutter, laser, or scalpel. To accomplish these steps, an initial step comprises inserting the distal end of a cutting tool into a bodily passage. Another step comprises navigating the distal end of the cutting tool to a point of treatment. The step of navigating the distal end of a cutting tool to a point of treatment can be accomplished using any suitable visualization technique, such as those described herein. Another step comprises cutting tissue from the bodily passage. Another step comprises removing the tissue from the bodily passage. Another step comprises removing the distal end of the cutting tool from the bodily passage.

An additional optional step comprises reducing the pressure of the second balloon after damaging the wall of the bodily passage to a pressure sufficient to provide contact between a portion of the first balloon and the passage wall. This step can be accomplished by removing a portion of the fluid from the inflation chamber until a desired pressure is reached.

While the various catheter configurations, steps, alternative steps, and optional steps have been described above with respect to treating the wall of a bodily passage, these catheter configurations, steps, alternative steps, and optional steps can be accomplished with respect to treating any suitable bodily passage including, but not limited to, a vein, artery, a sinus cavity, airway, and/or sinus passage and/or the wall and/or tissue of each of these bodily passages. In addition, these catheter configurations, steps, alternative steps, and optional steps can be used with respect to treating any suitable condition, including, but not limited to, an airway stricture, sinus cavity stricture, sinus passage stricture, vascular stricture, atherosclerosis, tracheal stenosis, sinus disease, or any other condition in which the catheters and/or methodologies described herein would be considered suitable.

The foregoing detailed description provides exemplary embodiments of the invention and includes the best mode for practicing the invention. The description and illustration of embodiments is intended only to provide examples of the invention, and not to limit the scope of the invention, or its protection, in any manner.

## Claims

1. A medical device for treating a bodily passage having a wall, said medical device comprising:
an elongate member (12) having an elongate member proximal end, an elongate member distal end, and a lengthwise axis, the elongate member defining an infusion lumen (26) and an inflation lumen (28);
a first balloon (14) attached to the elongate member and adapted to move between a deflated configuration and an inflated configuration, the first balloon comprising a first balloon wall defining an infusion chamber and at least one protuberance (52) extending radially outward from the first balloon wall, the at least one protuberance having a protuberance height (57), a protuberance length, and a protuberance outer surface (58), the infusion chamber in communication with the infusion lumen, the at least one protuberance defining a plurality of protuberance pores (56) in communication with the infusion chamber such that fluid introduced into the infusion chamber can pass through each protuberance pore of the plurality of protuberance pores, each pore of the plurality of protuberance pores having an opening defined on the protuberance outer surface; and
a second balloon (16) attached to the elongate member and disposed radially inward of the first balloon, the second balloon adapted to move between a deflated configuration and an inflated configuration and comprising a second balloon wall defining an inflation chamber in communication with the inflation lumen;
the at least one protuberance (52) extending radially outward from the first balloon wall to the protuberance outer surface (58), and each protuberance pore (56) of the plurality of protuberance pores extending through the protuberance height (57);
wherein the protuberance outer surface (58) is adapted to damage said wall of said bodily passage when the second balloon (16) is in the inflated configuration; and
wherein the plurality of protuberance pores is arranged along the protuberance length of the at least one protuberance relative to the lengthwise axis of the elongate member (12).

2. The medical device of claim 1, wherein a first protuberance pore of the plurality of protuberance pores comprises a first diameter;
wherein a second protuberance pore of the plurality of protuberance pores comprises a second diameter; and
wherein the second diameter is different than the first diameter.

3. The medical device of claim 2, wherein each protuberance pore in a first set of protuberance pores of the plurality of protuberance pores comprises a first diameter;
wherein each protuberance pore in a second set of protuberance pores of the plurality of protuberance pores comprises a second diameter; and
wherein the second diameter is different than the first diameter.

4. The medical device of claim 2, wherein the first balloon (14) comprises a first balloon proximal end and a first balloon distal end;
wherein a first protuberance pore of the plurality of protuberance pores comprises a first diameter and is disposed between the first balloon proximal end and the first balloon distal end;
wherein a second protuberance pore of the plurality of protuberance pores comprises a second diameter and is disposed between the first protuberance pore of the plurality of protuberance pores and the first balloon distal end;
wherein a third protuberance pore of the plurality of protuberance pores comprises a third diameter and is disposed between the second protuberance pore of the plurality of protuberance pores and the first balloon distal end;
wherein the third diameter is greater than the second diameter; and
wherein the second diameter is greater than the first diameter.

5. The medical device of any one of the preceding claims, wherein the first balloon wall defines at least one wall pore in communication with the infusion chamber such that fluid introduced into the infusion chamber can pass through the at least one wall pore.

6. The medical device of claim 5, wherein at least one wall pore comprises a plurality of wall pores (680,780).

7. The medical device of claim 6, wherein a first protuberance pore of the plurality of protuberance pores comprises a first diameter;
wherein a first wall pore of the plurality of wall pores comprises a second diameter; and
wherein the second diameter is different than the first diameter.

8. The medical device of claim 6, wherein each protuberance pore in a first set of protuberance pores of the plurality of protuberance pores comprises a first diameter;
wherein each wall pore in a first set of wall pores of the plurality of wall pores comprises a second diameter; and
wherein the second diameter is different than the first diameter.

9. The medical device of claim 6, wherein the first balloon (14) comprises a first balloon proximal end and a first balloon distal end;
wherein a first wall pore of the plurality of wall pores comprises a first diameter and is disposed between the first balloon proximal end and the first balloon distal end;
wherein a second wall pore of the plurality of wall pores comprises a second diameter and is disposed between the first wall pore of the plurality of wall pores and the first balloon distal end;
wherein a third wall pore of the plurality of wall pores comprises a third diameter and is disposed between the second wall pore of the plurality of wall pores and the first balloon distal end;
wherein the third diameter is greater than the second diameter; and
wherein the second diameter is greater than the first diameter.

## Patentansprüche

1. Medizinische Vorrichtung zum Behandeln eines Körperdurchgangs mit einer Wand, wobei die medizinische Vorrichtung umfasst:
ein längliches Element (12) mit einem proximalen Ende des länglichen Elements, einem distalen Ende des länglichen Elements, und einer Längsachse, wobei das längliche Element ein Infusionslumen (26) und ein Aufblaslumen (28) definiert;
einen ersten Ballon (14), der an dem länglichen Element befestigt ist und vorgesehen ist, um sich zwischen einer entleerten Konfiguration und einer aufgeblasenen Konfiguration zu bewegen, wobei der erste Ballon eine erste Ballonwand, die eine Infusionskammer definiert, und mindestens einen Vorsprung (52) umfasst, der sich von der ersten Ballonwand radial auswärts erstreckt, wobei der mindestens eine Vorsprung eine Vorsprunghöhe (57), eine Vorsprunglänge und eine Vorsprungaußenfläche (58) aufweist, wobei die Infusionskammer in Kommunikation mit dem Infusionslumen ist, der mindestens eine Vorsprung eine Vielzahl von Vorsprungporen (56) in Kommunikation mit der Infusionskammer definiert, so dass in die Infusionskammer eingebrachtes Fluid durch jede Vorsprungpore von der Vielzahl der Vorsprungporen hindurch gelangen kann, wobei jede Pore von der Vielzahl der Vorsprungporen eine Öffnung aufweist, die auf der Vorsprungaußenfläche definiert ist; und
einen zweiten Ballon (16), der an dem länglichen Element befestigt ist und radial einwärts des ersten Ballons befestigt ist, wobei der zweite Ballon vorgesehen ist, um sich zwischen einer entleerten Konfiguration und einer aufgeblasenen Konfiguration zu bewegen, und umfassend eine zweite Ballonwand, die eine Aufblaskammer in Kommunikation mit dem Aufblaslumen definiert;
wobei der mindestens eine Vorsprung (52) sich von der ersten Ballonwand radial auswärts zu der Vorsprungaußenfläche (58) erstreckt, und jede Vorsprungpore (56) von der Vielzahl der Vorsprungporen sich durch die Vorsprunghöhe (57) hindurch erstreckt;
wobei die Vorsprungaußenfläche (58) vorgesehen ist, um die Wand des Körperdurchgangs zu beschädigen, wenn sich der zweite Ballon (16) in der aufgeblasenen Konfiguration befindet; und
wobei die Vielzahl der Vorsprungporen entlang der Vorsprunglänge des mindestens einen Vorsprungs relativ zu der Längsachse des länglichen Elements (12) angeordnet ist.

2. Medizinische Vorrichtung nach Anspruch 1, wobei eine erste Vorsprungpore von der Vielzahl der Vorsprungporen einen ersten Durchmesser umfasst;
wobei eine zweite Vorsprungpore von der Vielzahl der Vorsprungporen einen zweiten Durchmesser umfasst; und
wobei der zweite Durchmesser sich von dem ersten Durchmesser unterscheidet.

3. Medizinische Vorrichtung nach Anspruch 2, wobei jede Vorsprungpore in einem ersten Satz von Vorsprungporen von der Vielzahl der Vorsprungporen einen ersten Durchmesser umfasst;
jede Vorsprungpore in einem zweiten Satz von Vorsprungporen von der Vielzahl der Vorsprungporen einen zweiten Durchmesser umfasst; und
wobei der zweite Durchmesser sich von dem ersten Durchmesser unterscheidet.

4. Medizinische Vorrichtung nach Anspruch 2, wobei der erste Ballon (14) ein proximales Ende des ersten Ballons und ein distales Ende des ersten Ballons umfasst;
eine erste Vorsprungpore von der Vielzahl der Vorsprungporen einen ersten Durchmesser umfasst und zwischen dem proximalen Ende des ersten Ballons und dem distalen Ende des ersten Ballons angeordnet ist;
wobei eine zweite Vorsprungpore von der Vielzahl der Vorsprungporen einen zweiten Durchmesser umfasst und zwischen der ersten Vorsprungpore von der Vielzahl der Vorsprungporen und dem distalen Ende des ersten Ballons angeordnet ist;
wobei eine dritte Vorsprungpore von der Vielzahl der Vorsprungporen einen dritten Durchmesser umfasst und zwischen der zweiten Vorsprungpore von der Vielzahl der Vorsprungporen und dem distalen Ende des ersten Ballons angeordnet ist;
wobei der dritte Durchmesser größer als der zweite Durchmesser ist; und
wobei der zweite Durchmesser größer als der erste Durchmesser ist.

5. Medizinische Vorrichtung nach einem der vorhergehenden Ansprüche, wobei die erste Ballonwand mindestens eine Wandpore in Kommunikation mit der Infusionskammer definiert, so dass in die Infusionskammer eingebrachtes Fluid durch die mindestens eine Wandpore hindurch gelangen kann.

6. Medizinische Vorrichtung nach Anspruch 5, wobei mindestens eine Wandpore eine Vielzahl von Wandporen (680, 780) umfasst.

7. Medizinische Vorrichtung nach Anspruch 6, wobei eine erste Vorsprungpore von der Vielzahl der Vorsprungporen einen ersten Durchmesser umfasst;
wobei eine erste Wandpore von der Vielzahl der Wandporen einen zweiten Durchmesser umfasst; und wobei der zweite Durchmesser sich von dem ersten Durchmesser unterscheidet.

8. Medizinische Vorrichtung nach Anspruch 6,
wobei jede Vorsprungpore in einem ersten Satz von Vorsprungporen von der Vielzahl der Vorsprungporen einen ersten Durchmesser umfasst;
jede Wandpore in einem ersten Satz von Wandporen von der Vielzahl der Wandporen einen zweiten Durchmesser umfasst; und
wobei der zweite Durchmesser sich von dem ersten Durchmesser unterscheidet.

9. Medizinische Vorrichtung nach Anspruch 6,
wobei der erste Ballon (14) ein proximales Ende des ersten Ballons und ein distales Ende des ersten Ballons umfasst;
wobei eine erste Wandpore von der Vielzahl der Wandporen einen ersten Durchmesser umfasst und zwischen dem proximalen Ende des ersten Ballons und dem distalen Ende des ersten Ballons angeordnet ist;
wobei eine zweite Wandpore von der Vielzahl der Wandporen einen zweiten Durchmesser umfasst und zwischen der ersten Wandpore von der Vielzahl der Wandporen und dem distalen Ende des ersten Ballons angeordnet ist;
wobei eine dritte Wandpore von der Vielzahl der Wandporen einen dritten Durchmesser umfasst und zwischen der zweiten Wandpore von der Vielzahl der Wandporen und dem distalen Ende des ersten Ballons angeordnet ist;
wobei der dritte Durchmesser größer als der zweite Durchmesser ist; und
wobei der zweite Durchmesser größer als der erste Durchmesser ist.

## Revendications

1. Dispositif médical pour traiter un passage corporel comportant une paroi, ledit dispositif médical comprenant :
un élément allongé (12) comportant une extrémité proximale d'élément allongé, une extrémité distale d'élément allongé et un axe longitudinal, l'élément allongé délimitant une lumière de perfusion (26) et une lumière de gonflage (28) ;
un premier ballonnet (14) fixé à l'élément allongé et apte à évoluer entre une configuration dégonflée et une configuration gonflée, le premier ballonnet comprenant une paroi de premier ballonnet délimitant une chambre de perfusion et au moins une protubérance (52) s'étendant radialement vers l'extérieur à partir de la paroi de premier ballonnet, ladite protubérance ayant une hauteur (57) de protubérance, une longueur de protubérance et une surface extérieure (58) de protubérance, la chambre de perfusion étant en communication avec la lumière de perfusion, ladite protubérance délimitant une pluralité de pores (56) de protubérance en communication avec la chambre de perfusion de telle sorte que du fluide introduit dans la chambre de perfusion peut passer à travers chaque pore de protubérance de la pluralité de pores de protubérance, chaque pore de la pluralité de pores de protubérance comportant une ouverture délimitée sur la surface extérieure de protubérance ; et
un second ballonnet (16) fixé à l'élément allongé et disposé radialement vers l'intérieur du premier ballonnet, le second ballonnet étant apte à évoluer entre une configuration dégonflée et une configuration gonflée et comprenant une paroi de second ballonnet délimitant une chambre de gonflage en communication avec la lumière de gonflage ;
ladite protubérance (52) s'étendant radialement vers l'extérieur de la paroi de premier ballonnet à la surface extérieure (58) de protubérance, et chaque pore (56) de protubérance de la pluralité de pores de protubérance traversant la hauteur (57) de protubérance,
dans lequel la surface extérieure (58) de protubérance est apte à endommager ladite paroi dudit passage corporel quand le second ballonnet (16) est en configuration gonflée ; et
dans lequel la pluralité de pores de protubérance est agencée le long de la longueur de protubérance de ladite protubérance par rapport à l'axe longitudinal de l'élément allongé (12).

2. Dispositif médical selon la revendication 1, dans lequel un premier pore de protubérance de la pluralité de pores de protubérance comprend un premier diamètre ;
dans lequel un deuxième pore de protubérance de la pluralité de pores de protubérance comprend un deuxième diamètre ; et
dans lequel le deuxième diamètre est différent du premier diamètre.

3. Dispositif médical selon la revendication 2, dans lequel chaque pore de protubérance dans une première série de pores de protubérance de la pluralité de pores de protubérance comprend un premier diamètre ;
chaque pore de protubérance dans une deuxième série de pores de protubérance de la pluralité de pores de protubérance comprend un deuxième diamètre ; et
dans lequel le deuxième diamètre est différent du premier diamètre.

4. Dispositif médical selon la revendication 2, dans lequel le premier ballonnet (14) comprend une extrémité proximale de premier ballonnet et une extrémité distale de premier ballonnet ;
dans lequel un premier pore de protubérance de la pluralité de pores de protubérance comprend un premier diamètre et est disposé entre l'extrémité proximale de premier ballonnet et l'extrémité distale de premier ballonnet ;
dans lequel un deuxième pore de protubérance de la pluralité de pores de protubérance comprend un deuxième diamètre et est disposé entre le premier pore de protubérance de la pluralité de pores de protubérance et l'extrémité distale de premier ballonnet ;
dans lequel un troisième pore de protubérance de la pluralité de pores de protubérance comprend un troisième diamètre et est disposé entre le deuxième pore de protubérance de la pluralité de pores de protubérance et l'extrémité distale de premier ballonnet ;
dans lequel le troisième diamètre est supérieur au deuxième diamètre ; et
dans lequel le deuxième diamètre est supérieur au premier diamètre.

5. Dispositif médical selon l'une quelconque des revendications précédentes, dans lequel la paroi de premier ballonnet délimite au moins un pore de paroi en communication avec la chambre de perfusion de telle sorte que du fluide introduit dans la chambre de perfusion peut passer à travers ledit pore de paroi.

6. Dispositif médical selon la revendication 5, dans lequel au moins un pore de paroi comprend une pluralité de pores (680, 780) de paroi.

7. Dispositif médical selon la revendication 6, dans lequel un premier pore de protubérance de la pluralité de pores de protubérance comprend un premier diamètre ;
dans lequel un premier pore de paroi de la pluralité de pores de paroi comprend un deuxième diamètre ; et
dans lequel le deuxième diamètre est différent du premier diamètre.

8. Dispositif médical selon la revendication 6, dans lequel chaque pore de protubérance dans une première série de pores de protubérance de la pluralité de pores de protubérance comprend un premier diamètre ;
dans lequel chaque pore de paroi dans une première série de pores de paroi de la pluralité de pores de paroi comprend un deuxième diamètre ; et
dans lequel le deuxième diamètre est différent du premier diamètre.

9. Dispositif médical selon la revendication 6, dans lequel le premier ballonnet (14) comprend une extrémité proximale de premier ballonnet et une extrémité distale de premier ballonnet ;
dans lequel un premier pore de paroi de la pluralité de pores de paroi comprend un premier diamètre et est disposé entre l'extrémité proximale de premier ballonnet et l'extrémité distale de premier ballonnet ;
dans lequel un deuxième pore de paroi de la pluralité de pores de paroi comprend un deuxième diamètre et est disposé entre le premier pore de paroi de la pluralité de pores de paroi et l'extrémité distale de premier ballonnet ;
dans lequel un troisième pore de paroi de la pluralité de pores de paroi comprend un troisième diamètre et est disposé entre le deuxième pore de paroi de la pluralité de pores de paroi et l'extrémité distale de premier ballonnet ;
dans lequel le troisième diamètre est supérieur au deuxième diamètre ; et
dans lequel le deuxième diamètre est supérieur au premier diamètre.
